# EUROPÄISCHE PATENTSCHRIFT

(11) **EP 0 382 862 B1**
(45) Veröffentlichungstag und Bekanntmachung des Hinweises auf die Patenterteilung: **28.04.1993**
(21) Anmeldenummer: 89102459.8
(22) Anmeldetag: 13.02.1989
(51) Int. Cl.: A61B 6/03

(54) **Computertomograph**
Computerized tomography apparatus
Appareil de tomographie par ordinateur

(43) Veröffentlichungstag der Anmeldung: 22.08.1990
(73) Patentinhaber: SIEMENS AKTIENGESELLSCHAFT, 80333 München (DE)
(72) Erfinder: Schwierz, Günter, Dr.rer.nat., D-8524 Neunkirchen (DE)

(56) Entgegenhaltungen:
- EP-A- 0 001 640
- DE-A- 2 729 833
- US-A- 4 206 362

## Beschreibung

Computertomographen erlauben die Erzeugung von Schnittbildern endlich dicker Schichten des Patienten, die im wesentlichen senkrecht zur Körperlängsachse des Patienten orientiert sind. Häufig ist die Darstellung eines ausgedehnten Organs und eines die Schichtdicke um ein Vielfaches übersteigenden Volumens erforderlich. Dazu muß eine unter Umständen umfangreiche Serie von parallelen Schnittbildern aufgenommen werden. Dabei kann ein kontinuierlicher oder diskontinuierlicher Patientenvorschub erfolgen.

Bei vorgegebener Schichtdicke und damit festgelegtem maximalem Vorschubinkrement sowie vertretbarer Gesamtaufnahmezeit wird das darstellbare Volumen durch die endliche Wärmekapazität des Röntgenstrahlers begrenzt.

Praktisch alle bekannten Computertomographen sind in einem Mode betreibbar, bei dem üblichen Projektionsbildern entsprechende Übersichtsbilder aufgenommen werden. Ein solches Übersichtsbild erleichtert dem Radiologen die Vorwahl des darzustellenden Volumens. Die Festlegung der Aufnahmeparameter "Schichtdicke, Schichtvorschub und Bilddosis" hat dann aber wieder unter Berücksichtigung der endlicher Wärmekapazität des Röntgenstrahlers zu erfolgen.

In der US-A-4 206 362 ist ein Computertomograph mit nicht drehbarem Detektorring und mit zwei Röntgenstrahlern beschrieben, so daß zwei angrenzende Schichten gleichzeitig abgetastet werden. In der EP-A-0 001 640 ist eine radiologische Einrichtung beschrieben, bei der ein Rechner die Aufnahmeparameter optimiert und vor Aufnahmebeginn den jeweils optimalen Fokus wählt. Schließlich ist in der DE-A-27 29 833 ein Computertomograph mit drei Röntgenstrahlern und drei mitgedrehten, teilkreisförmigen Strahlendetektoren beschrieben.

Der Erfindung liegt die Aufgabe zugrunde, einen Computertomographen so auszubilden, daß die Aufnahme eines im Vergleich zum Stand der Technik großen Volumens des Untersuchungsobjektes möglich ist, ohne daß die Grenze der Wärmespeicherfähigkeit des abtastenden Röntgenstrahlers erreicht wird.

Diese Aufgabe ist erfindungsgemäß gelöst durch die Merkmale des Patentanspruches 1.

Bei dem erfindungsgemäßen Computertomographen kann zur Erzeugung der Detektordaten der Detektorelemente des Detektorringes zunächst ein Röntgenstrahler eingeschaltet werden. Wird die Grenze der Wärmekapazität des Röntgenstrahlers erreicht, so wird auf einen anderen Röntgenstrahler umgeschaltet. Demgemäß ist ein großes Volumen schnell abtastbar.

Wenn der Drehrahmen mit den Röntgenstrahlern den Detektorring umschließt, also die Röntgenstrahler mit ihren Foken auf einem Kreis bewegt werden, der außerhalb des Detektorringes liegt, ist der Mode zur Anfertigung von Übersichtsaufnahmen mit geringen Kosten für die Meßkanäle realisierbar. Durch die Möglichkeit der Schichtvorselektion aufgrund dieses Mode und der ausreichenden Wärmekapazität der Röntgenstrahler eignet sich der erfindungsgemäße Computertomograph hervorragend für alle Aufnahmeanforderungen, von der Aufnahme einzelner dünner Schichten bis hin zur Darstellung großer Volumen.

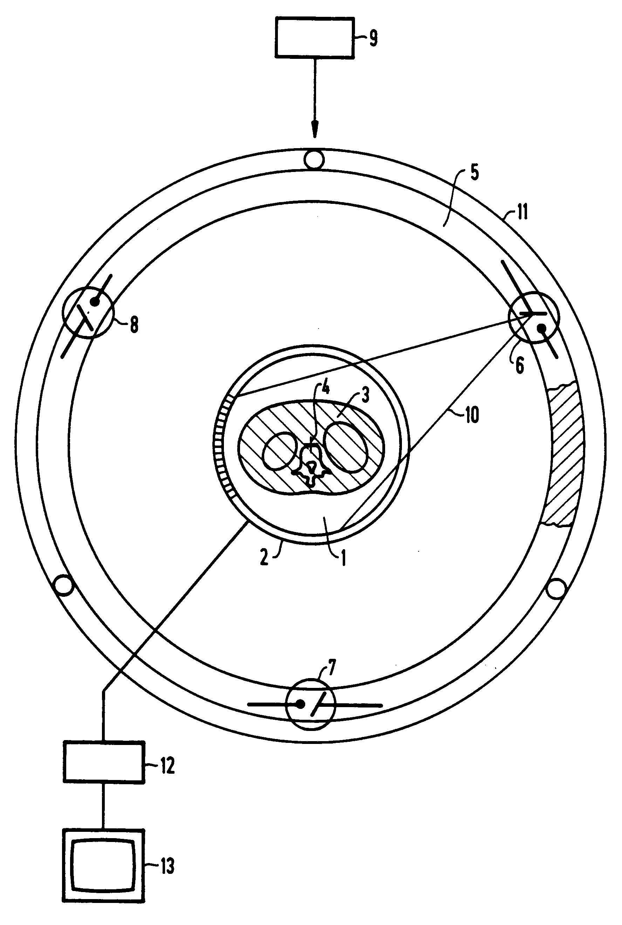

Die Erfindung ist nachfolgend anhand eines in der Zeichnung dargestellten Ausführungsbeispieles näher erläutert.

In der Zeichnung ist ein Computertomograph mit einem eine Meßöffnung 1 umschließenden, aus einer Reihe von Detektorelementen bestehenden, nicht drehbaren Detektorring 2 dargestellt. In der Meßöffnung 1 liegt ein Untersuchungsobjekt 3, das im Querschnitt dargestellt ist. Die Achse des Detektorringes 2 ist mit 4 bezeichnet.

Zur Abtastung des Untersuchungsobjektes 3 mit Hilfe von fächerförmigen Röntgenstrahlenbündeln sind auf einem Drehrahmen 5 drei Röntgenstrahler 6, 7, 8 angeordnet, die von einem Röntgengenerator 9 gespeist werden. Jeweils einer der Röntgenstrahler 6 bis 8 wird eingeschaltet. Bei dem Beispiel ist der Röntgenstrahler 6 eingeschaltet, dessen Röntgenstrahlenbündel mit 10 bezeichnet ist. Zur Änderung der Richtung, unter der das Untersuchungsobjekt 3 abgetastet wird, wird der Drehrahmen 5 um die Achse 4 in einem Lagerring 11 gedreht. Der Drehrahmen 5 umschließt den Detektorring 2, so daß sich die Foken der Röntgenstrahler 6 bis 8 auf einem Kreis bewegen, der außerhalb des Detektorringes 2 liegt. Damit das jeweils eingeschaltete Röntgenstrahlenbündel 10 in Strahlenrichtung gesehen vor dem Patienten seitlich am Detektorring 2 vorbei verläuft, also noch nicht Detektorelemente trifft, kann der Detektorring 2 in bekannter Weise taumelnd gelagert sein und synchron mit dem Drehrahmen 5 entsprechend so verstellt werden, daß das Röntgenstrahlenbündel 10 erst nach dem Austritt aus dem Untersuchungsobjekt 3 auf den Detektorelementen auftrifft. Eine andere Möglichkeit besteht darin, das jeweils eingeschaltete Röntgenstrahlenbündel 10 unter einem von 90° abweichenden Winkel zur Achse 4 auszurichten.

Ein Rechner 12 erzeugt aus den Detektordaten die Verteilung der Schwächungskoeffizienten in einer Bildmatrix und bewirkt deren bildliche Wiedergabe auf einem Sichtgerät 13.

Es ist möglich, die Halterung für die Röntgenstrahler 6 bis 8 auf dem Drehrahmen 5 so auszubilden, daß der Computertomograph nach Wunsch des Benutzers wahlweise mit einem oder mit mehreren, gleichmäßig auf dem Drehrahmen 5 verteilten Röntgenstrahlern bestückt werden kann.

Die Abtastung des Untersuchungsobjektes 3 erfolgt in der Weise, daß dann, wenn die Grenze der Wärmekapazität des jeweils eingeschalteten Röntgenstrahlers 6 bis 8 erreicht wird, auf einen anderen, vorher nicht eingeschalteten Röntgenstrahler umgeschaltet wird.

## Patentansprüche

1. Computertomograph mit nicht drehbarem, aus einer Reihe von Detektorelementen bestehendem, Detektorring (2) mit folgenden Merkmalen:
es sind mehrere, jeweils ein fächerförmiges, auf dem Detektorring (2) auftreffendes Röntgenstrahlenbündel (10) aussendende Röntgenstrahler (6, 7, 8) vorhanden;
die Röntgenstrahler (6, 7, 8) sind zur Abtastung eines Untersuchungsobjektes (3) unter verschiedenen Richtungen auf einem gemeinsamen Drehrahmen (5) gelagert;
ein Rechner (12) erzeugt aus Ausgangsignalen der Detektorelemente die Verteilung der Schwächungskoeffizienten in einer Bildmatrix und bewirkt deren bildliche Wiedergabe; dadurch gekennzeichnet, daß eine Einrichtung vorgesehen ist, die jeweils einen der Röntgenstrahler einschaltet und dann, wenn die Grenze der Wärmekapazität des jeweils eingeschalteten Röntgenstrahlers (6, 7, 8) erreicht ist, auf einen anderen, vorher nicht eingeschalteten Röntgenstrahler (6, 7, 8) umschaltet.

2. Computertomograph nach Anspruch 1, **dadurch gekennzeichnet,** daß die Röntgenstrahler (6, 7, 8) gleichmäßig auf dem Drehrahmen (5) angeordnet sind.

3. Computertomograph nach Anspruch 2, **dadurch gekennzeichnet,** daß drei Röntgenstrahler (6, 7, 8) vorhanden sind.

4. Computertomograph nach einem der Ansprüche 1 bis 3, **dadurch gekennzeichnet,** daß der Drehrahmen (5) den Detektorring (2) umschließt.

## Claims

1. A computer tomography apparatus with a detector ring (2) which is not able to rotate and consisting of a series of detector elements, with the following features:
- several X-ray sources (6,7,8) emitting in each case a fan-shaped X-ray beam (10) striking the detector ring (2) are present;
- the X-ray sources (6,7,8) for scanning an object under examination (3) from various directions are supported on a common rotating frame (5);
- a computer (12) produces from output signals of the detector elements the distribution of the linear attenuation coefficients in an image matrix and effects the pictorial reproduction thereof; characterised in that a device is provided, which connects in each case one of the X-ray sources,
- and then when the limit of the heat capacity of the respective connected X-ray source (6,7,8) is reached, switches over to another X-ray source (6,7,8), not connected previously.

2. A computer tomography apparatus according to claim 1 characterised in that the X-ray sources (6,7,8) are arranged evenly on the rotating frame (5).

3. A computer tomography apparatus according to claim 2, characterised in that three X-ray sources (6,7,8) are present.

4. A computer tomography apparatus according to one of claims 1 to 3, characterised in that the rotating frame (5) surrounds the detector ring (2).

## Revendications

1. Tomodensitomètre comportant un anneau non rotatif de détecteurs (2), qui est constitué par une série d'éléments détecteurs, présentant les caractéristiques suivantes :
il est prévu plusieurs émetteurs radiologiques (6,7,8), qui émettent chacun un faisceau (10) de rayons X en forme d'éventail, qui rencontre l'anneau de détecteurs (2); les émetteurs radiologiques (6,7,8) sont montés sur un cadre rotatif commun (5) de manière à balayer un objet d'examen (3) dans différentes directions; un calculateur (12) produit, à partir des signaux de sortie des éléments détecteurs, la répartition des coefficients d'affaiblissement sous la forme d'une matrice d'image et reproduisent cette matrice sous la forme d'une image; caractérisé par le fait qu'il est prévu un dispositif qui branche respectivement l'un des émetteurs radiologiques et, lorsque la limite de la capacité calorifique de l'émetteur radiologique (6,7,8) respectivement branché est atteinte, exécute une commutation sur un autre émetteur radiologique (6,7,8), non branché auparavant.

2. Tomodensitomètre suivant la revendication 1, ccaractérisé par le fait que les émetteurs radiologiques (6,7,8) sont disposés d'une manière uniforme sur le cadre rotatif (5).

3. Tomodensitomètre suivant la revendication 2, caractérisé par le fait qu'il est prévu trois émetteurs radiologiques (6,7,8).

4. Tomodensitomètre suivant l'une des revendications 1 à 3, caractérisé par le fait que le cadre rotatif (5) entoure l'anneau de détecteurs (2).
